# EUROPEAN PATENT SPECIFICATION

(11) **EP 4 438 068 B1**
(45) Date of publication and mention of the grant of the patent: **16.09.2026**
(21) Application number: 24159091.8
(22) Date of filing: 22.02.2024
(51) Int. Cl.: A61L 27/22, A61L 27/24, A61L 27/38, A61L 27/52

(54) **GEL PRECURSOR COMPOSITION, METHOD FOR PRODUCING GEL, AND METHOD FOR ACTIVATING ENZYME**
GELVORLÄUFERZUSAMMENSETZUNG, VERFAHREN ZUR HERSTELLUNG EINES GELS UND VERFAHREN ZUR ENZYMAKTIVIERUNG
COMPOSITION DE PRÉCURSEUR DE GEL, PROCÉDÉ DE PRODUCTION DE GEL ET PROCÉDÉ D'ACTIVATION D'ENZYME

(30) Priority: 23.03.2023 JP 2023046165
(43) Date of publication of application: 02.10.2024
(73) Proprietor: Ricoh Company, Ltd., Tokyo 143-8555 (JP)
(72) Inventor: ARAI, Daisuke, Tokyo, 143-8555 (JP); MATSUMOTO, Takahiko, Tokyo, 143-8555 (JP); NONOYAMA, Yusuke, Toyko, 143-8555 (JP); YAGINUMA, Hidekazu, Tokyo, 143-8555 (JP)
(74) Representative: Marks & Clerk LLP

(56) References cited:
- US-A1- 2024 042 101
- PENG KE ET AL: "Light manipulation for fabrication of hydrogels and their biological applications", ACTA BIOMATERIALIA, ELSEVIER, AMSTERDAM, NL, vol. 137, 9 October 2021 (2021-10-09), pages 20 - 43, XP086897484, ISSN: 1742-7061, [retrieved on 20211009], DOI: 10.1016/J.ACTBIO.2021.10.003
- VALERIA NELE ET AL: "Tailoring Gelation Mechanisms for Advanced Hydrogel Applications", ADVANCED FUNCTIONAL MATERIALS, WILEY - V C H VERLAG GMBH & CO. KGAA, DE, vol. 30, no. 42, 18 August 2020 (2020-08-18), pages n/a, XP072410098, ISSN: 1616-301X, DOI: 10.1002/ADFM.202002759
- FEDERICO I ROSELL ET AL: "Photochemical reagents for the study of metalloproteins by flash photolysis", COORDINATION CHEMISTRY REVIEWS, ELSEVIER SCIENCE, AMSTERDAM, NL, vol. 255, no. 7, 17 November 2010 (2010-11-17), pages 737 - 756, XP028168178, ISSN: 0010-8545, [retrieved on 20101125], DOI: 10.1016/J.CCR.2010.11.025
- MOHAMED MOHAMED ALAA ET AL: "Stimuli-responsive hydrogels for manipulation of cell microenvironment: From chemistry to biofabrication technology", PROGRESS IN POLYMER SCIENCE, PERGAMON PRESS, OXFORD, GB, vol. 98, 12 August 2019 (2019-08-12), XP085886739, ISSN: 0079-6700, [retrieved on 20190812], DOI: 10.1016/J.PROGPOLYMSCI.2019.101147
- TOMPA P ET AL: "Frequency decoding of fast calcium oscillations by calpain", CELL CALCIUM, ELSEVIER, AMSTERDAM, NL, vol. 29, no. 3, 25 May 2002 (2002-05-25), pages 161 - 170, XP029575414, ISSN: 0143-4160, DOI: 10.1054/CECA.2000.0179

## Description

### BACKGROUND OF THE INVENTION

### Field of the Invention

The present disclosure relates to a gel precursor composition, a method for producing a gel, and a method for activating an enzyme.

### Description of the Related Art

In recent years, research and development related to human regenerative medicine has been advanced. One method for regenerative medicine is being attempted, in which method cells of a patient's own, human iPS cells, or heterologous cells are cultured in vitro, and tissues containing the cells cultured are transplanted to treat a damaged site of the patient or provide a transplant as a substitute for a malfunctioning organ.

Patent Literature 1 discloses a process for gelation, wherein the process includes the steps of: a) providing a mixture including a liposome and a gel precursor; wherein the liposome encapsulates a payload that is capable of inducing gelation of the gel precursor; b) applying ultrasound to the mixture; to trigger the release of the payload from the liposome and induce gelation of the gel precursor. As a method in which the payload induces the gelation, Patent Literature 1 discloses a method in which the payload acts directly on the precursor to induce gelation, or the payload acts indirectly on the precursor to induce gelation, for example, by activation of an enzyme. Peng et al., Acta Biomater., 2022, 137, 20-43 relates to light manipulation for fabrication of hydrogels and their biological applications.

### SUMMARY OF THE INVENTION

An object of the present disclosure is to provide a gel precursor composition causative of no apprehension about exhibiting cytotoxicity and a method for producing a gel causative of no apprehension about exhibiting cytotoxicity, using the gel precursor composition.

To achieve the above-described object, one aspect of the present invention is a gel precursor composition including: a biocompatible polymer; a crosslinking agent to be activated by a specific substance; and a photoresponsive compound bound to the specific substance. The biocompatible polymer is a material derived from an organism. The crosslinking agent is a calcium-dependent enzyme that has any activity or no activity, depending on the calcium-ion concentration. The specific substance is a calcium ion. The photoresponsive compound is caged calcium. The biocompatible polymer is a polymer that undergoes temperature-responsive sol-gel transition.

The present disclosure can provide a gel precursor composition causative of no apprehension about exhibiting cytotoxicity and a method for producing a gel causative of no apprehension about exhibiting cytotoxicity, using the gel precursor composition.

### BRIEF DESCRIPTION OF THE DRAWINGS

FIG. 1 is a schematic diagram illustrating one aspect in which an uncrosslinked polymer contained in a gel precursor composition is crosslinked to produce a gel;
FIG. 2 is an explanatory diagram of the mechanism of action by which an uncrosslinked polymer is crosslinked to gelate;
FIG. 3 is a schematic diagram illustrating one aspect in which, using a photomask member, an uncrosslinked polymer contained in a gel precursor composition is crosslinked to produce a gel;
FIG. 4 is a diagram illustrating an example of a gel obtained by gelation of a gel precursor composition utilizing the photomask member illustrated in FIG. 3, and having desired holes;
FIG. 5 is a schematic view illustrating Example of gelation of a gel precursor composition;
FIG. 6 is a view illustrating a gelatin hydrogel obtained in Example;
FIG. 7 is a schematic diagram of a process in which a gel having an arbitrary three-dimensional shape is produced by irradiating a gel precursor composition with light; and
FIG. 8 is a view illustrating a gel having a hollow shape.

### DESCRIPTION OF THE EMBODIMENTS

A gel precursor composition according to the present invention includes: a biocompatible polymer; a crosslinking agent to be activated by a specific substance; and an active-energy-line-responsive compound bound to the specific substance. The biocompatible polymer is a material derived from an organism. The crosslinking agent is a calcium-dependent enzyme that has any activity or no activity, depending on the calcium-ion concentration. The specific substance is a calcium ion. The active-energy-line-responsive compound is caged calcium. The biocompatible polymer is a polymer that undergoes temperature-responsive sol-gel transition.

A method for producing a gel according to the present invention includes irradiating the gel precursor composition with an active energy line to allow the gel precursor composition to gelate, the active energy line having the capability to cause the active-energy-line-responsive compound to release the specific substance into a system. A method for activating an enzyme according to the present disclosure is a method for activating an enzyme using a caged compound. Each component will be described below.

### (Active Energy Line)

The active energy line is not particularly limited as long as an active-energy-line-responsive compound can react with the active energy line to release the specific substance into a system. The active energy line is typically light such as visible light, ultraviolet rays, or infrared rays, and is, for example, an energy line such as an electron beam. Hereinafter, the present disclosure describes particularly an aspect in which light is used as a typical example, but use of an active energy line other than light is also encompassed in the present invention.

### (Biocompatible Polymer)

The biocompatible polymer is not particularly limited, and is a polymer that is biocompatible and can be crosslinked by a crosslinking agent. In the present invention, a biocompatible polymer yet to be crosslinked by a crosslinking agent is also referred to as an uncrosslinked polymer. A crosslinked biocompatible polymer is also referred to as a crosslinked polymer. The biocompatible polymer is a material derived from an organism. The species of the organism is not particularly limited. The species is, for example, mammalians, birds, reptiles, amphibians, fishes, insects, plants, and fungi, and can be suitably selected, for example, in accordance with the use of the gel precursor composition. In one preferable aspect, the biocompatible polymer is an extracellular matrix.

The biocompatible polymer differs depending on the kind of the crosslinking agent. The polymer is, for example, a protein from the viewpoint of the capability to easily form a gel by crosslinking, and is preferably at least one polymer selected from gelatin and collagen. The biocompatible polymer of one kind may be used singly, or the biocompatible polymers of two or more kinds may be used.

The biocompatible polymer is a polymer that undergoes temperature-responsive sol-gel transition. The biocompatible polymer that undergoes temperature-responsive sol-gel transition makes it possible that, for example, a gel precursor composition is allowed to gelate under temperature conditions which cause a biocompatible polymer to gelate, and that, after the gelation terminates, temperature conditions which cause the biocompatible polymer to solate are provided. Producing a gel under such conditions makes it possible that, in the gelation, the gel precursor composition has a sufficient physical strength, and that, after the gelation, the gel and the biocompatible polymer that has not gelated are separated easily.

### (Crosslinking Agent to be Activated by Specific Substance)

The crosslinking agent to be activated by the specific substance is not particularly limited, and can be a crosslinking agent that does not crosslink the biocompatible polymer before being activated by the specific substance, and acts as a crosslinking agent for the biocompatible polymer after being activated by the specific substance.

In the present disclosure, the specific substance is a calcium ion. The crosslinking agent to be activated by the specific substance is an enzyme from the viewpoint of the biocompatibility of the gel precursor composition. Examples of the enzyme include an enzyme derived from an organism. In the present disclosure, an enzyme contained in the gel precursor composition, and not activated by the specific substance is also referred to as an inactive enzyme. An enzyme activated by the specific substance is also referred to as an activated enzyme. The crosslinking agent is an enzyme, because, in such a case, the possibility of exhibiting cytotoxicity is particularly low while the method for producing a gel is performed, and hence, the method for producing a gel can be performed in a biogenic environment. The crosslinking agent to be activated by a specific substance is a calcium-dependent enzyme that has any activity or no activity, depending on the calcium ion concentration.

Examples of the calcium-dependent enzyme include transglutaminase that has any activity or no activity depending on the calcium ion concentration (hereinafter also referred to as calcium-dependent transglutaminase). Calcium-dependent transglutaminase can easily crosslink each of the proteins specifically enumerated in the above-described section on the biocompatible polymer. It is known that calcium-dependent transglutaminase in an activated state can be crosslinked, via an isopeptide bond, with a protein having a glutamine residue and a lysine residue. A primary amine instead of a lysine residue affords the crosslinkability. The crosslinking agent of one kind to be activated by a specific substance may be used singly, or the agents of two or more kinds may be used.

### (Photoresponsive Compound)

The photoresponsive compound is a photoresponsive compound that is bound to the specific substance. The photoresponsive compound can be a compound that is, for example, degraded and/or changed in the stereostructure by light-irradiation, and releases the specific substance into a system, thus enabling the crosslinking agent to be activated. Among the photoresponsive compounds in the present disclosure, a photoresponsive compound that is degraded by light-irradiation, and releases the specific substance system, thus enabling the crosslinking agent to be activated is also referred to as a photodegradable compound. The photoresponsive compound is a photodegradable compound. In a case where the crosslinking agent is an enzyme in the present disclosure, the specific substance is also referred to as an enzyme-activating substance.

From the viewpoint of the capability to easily release a specific substance into a system, the photoresponsive compound is a photodegradable compound, for example, a caged compound, and more specifically caged calcium. A caged compound can be a compound that has the specific substance incorporated in the compound by binding or encompassment, and can release the specific substance into a system by light-irradiation. For example, the caged calcium can be a compound that has a calcium ion incorporated in the compound by binding or encompassment, and can release the calcium ion into a system by light-irradiation.

The caged calcium that can be used is calcium bound to a caged reagent. Specific examples include calcium-bound DM-NITROPHEN, calcium-bound DMNP-EDTA, calcium-bound NP-EGTA, calcium-bound NDBF-EGTA, and calcium-bound azid-1. The photoresponsive compound of one kind may be used singly, or the photoresponsive compounds of two or more kinds may be used.

### (Gel Precursor Composition)

A gel precursor composition according to the present invention includes: a biocompatible polymer; a crosslinking agent to be activated by a specific substance; and a photoresponsive compound bound to the specific substance.

The gel precursor composition may contain another component. The another component can be suitably selected depending on the gel precursor composition, the use of a gel obtained from the gel precursor composition, the production conditions for a gel, and the like. For example, the gel precursor composition containing, besides the photoresponsive compound, a specific substance at a concentration not causative of activating a crosslinking agent can be made such that the gel precursor composition, if left as it is, does not undergo crosslinking, but can be produced into a gel by allowing a small amount of the photoresponsive compound to release the specific substance into a system by light-irradiation. The amount of each of the components contained in the gel precursor composition differs depending on the kind of each component, the use of the gel, the production conditions, and the like, and can be set suitably.

The gel precursor composition may contain at least one selected from a buffer and a pH adjustor. For example, it is generally known that the activity of an enzyme is influenced by a pH. Containing such a component enables the gel precursor composition to be adjusted to a desired pH.

A method for producing a gel precursor composition is not particularly limited. The gel precursor composition can be obtained by mixing: a biocompatible polymer; a crosslinking agent to be activated by a specific substance; and a photoresponsive compound bound to the specific substance. In the production of the gel precursor composition, a solvent may be used. The solvent is preferably water.

### (Method for Producing Gel)

A method for producing a gel according to the present invention includes irradiating the gel precursor composition with light to allow the gel precursor composition to gelate, the light having the capability to cause the photoresponsive compound to release the specific substance into a system. This process can yield a gel. In a case where the photoresponsive compound is the photodegradable compound, the light having the capability to cause the photoresponsive compound to release the specific substance into a system means light that degrades the photodegradable compound, and releases the specific substance into a system.

In the gelation process, the temperature of the gel precursor composition is preferably controlled. In particular, in a case where an enzyme is used as a crosslinking agent, the process is usually performed at a temperature that enables the characteristics of the enzyme to be exerted. In a case where the biocompatible polymer undergoes temperature-responsive sol-gel transition, performing the process with the polymer in a gelated state can enhance the strength of the gel precursor composition itself.

In one preferable aspect, the gelation process is performed in the presence of at least one selected from a buffer and a pH adjustor. Performing the process in the presence of such a component enables the process to be performed under desired pH conditions. The at least one selected from a buffer and a pH adjustor may be contained in the gel precursor composition, or may be used as a component other than the gel precursor composition in the gelation process.

In one preferable aspect, the gelation process is followed by adding at least one selected from: an inhibitor for the crosslinking agent; and a chelator for the specific substance. In the gelation process, the crosslinking agent activated advances the polymerization of the biocompatible polymer, but from the viewpoint of micro-molding or the like, the crosslinking agent activated is desirably inactivated at the stage where the desired gelation is achieved. Accordingly, in one aspect, an inhibitor for the crosslinking agent is used to inhibit the function characteristic of the crosslinking agent, or the specific substance is protected by a chelator, so that the interaction with the crosslinking agent is eliminated, and the crosslinking agent is inactivated.

The method for producing a gel does not involve preparing a mold each time a gel is produced. The method allows the production of a three-dimensional gel with high resolution and accuracy, and has excellent economical efficiency. The gel obtained can achieve a capability such as the retention of cell function or biodegradability, and thus, a method for producing a gel according to the present invention is extremely useful.

One embodiment of the present disclosure includes a gel obtained from the gel precursor composition. The gel is produced from a biocompatible polymer as a raw material, and thus, has excellent biocompatibility, and can be suitably used particularly in uses for organisms. Examples of uses of a gel include a cell culture base material and a therapeutic base material. That is, in one embodiment, the present disclosure includes: a gel that is obtained from the gel precursor composition, and is to be used for a cell culture base material or a therapeutic base material; and a cell culture base material or a therapeutic base material that contains a gel obtained from the gel precursor composition. In one embodiment, the present disclosure includes a gel precursor composition for producing a gel to be used for a cell culture base material or a therapeutic base material. That is, the gel precursor composition and the gel obtained can be utilized for a cell culture for drug development and a medical supply for regenerative medicine or the like. Examples of the gel include a hydrogel.

### (Method for Activating Enzyme Using Caged Compound)

As described above, the present disclosure includes a method for activating an enzyme using a caged compound. A method for activating an enzyme has been conventionally studied, and the present disclosure presents a method for activating an enzyme using a caged compound. One aspect of the activating method is, for example, a method for activating an enzyme, the method including a process in which light capable of degrading the caged compound is radiated in the presence of the enzyme and the caged compound. One example of the activating method is a method for activating a calcium-dependent enzyme using caged calcium. A more specific aspect is, for example, a method for activating a calcium-dependent enzyme, the method including a process in which light capable of degrading the caged calcium is radiated in the presence of the calcium-dependent enzyme and the caged calcium. The method for activating an enzyme may be performed as part of the above-described method for producing a gel.

Below, the gel precursor composition and an example of the method for producing a gel will be further described with reference to the drawings.

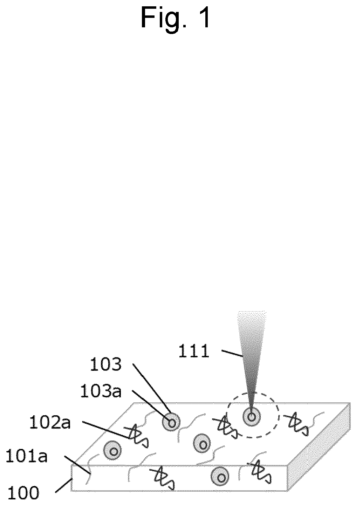

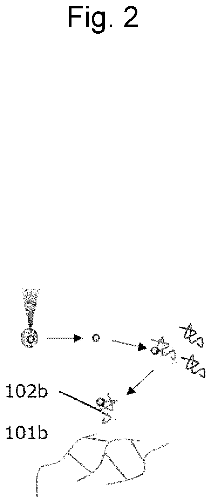

FIG. 1 is a schematic diagram of one aspect in which an uncrosslinked polymer contained in a gel precursor composition is crosslinked to produce a gel. A gel precursor composition 100 contains an uncrosslinked polymer 101a, an inactive enzyme 102a, and a caged compound 103. FIG. 2 is an explanatory diagram of the mechanism of action by which the uncrosslinked polymer 101a is crosslinked to gelate.

The gel precursor composition 100 may be retained in the form of liquid in a container, or may be in the form of a gel capable of maintaining the shape. The inactive enzyme 102a is activated and changed to an active enzyme 102b when the enzyme-activating substance 103a reaches a given or higher concentration. The active enzyme 102b crosslinks the uncrosslinked polymers 101a to generate a crosslinked polymer 101b, thus forming a gel.

The caged compound 103 is bound to the enzyme-activating substance 103a. The concentration of the enzyme-activating substance 103a in the gel precursor composition 100 (the concentration of the enzyme-activating substance 103a neither bound to nor encompassed in the caged compound 103) is adjusted not to exceed the threshold at which the enzyme is activated.

The caged compound 103 is irradiated with light at a given or larger amount of irradiation, so that a protecting group bound to the enzyme-activating substance 103a is degraded to release the enzyme-activating substance 103a. In FIG. 1, a laser beam 111 is illustrated as an example of radiated light.

In the area irradiated with the laser beam 111, the concentration of the enzyme-activating substance 103a rises locally, and in and near the irradiated area, the inactive enzyme 102a is changed to the active enzyme 102b. This active enzyme 102b locally crosslinks the uncrosslinked polymer 101a, which thus becomes the crosslinked polymer 101b. Thus, the gel precursor composition 100 can gelate in and near the area irradiated with the laser beam.

A laser beam can have a spot diameter reduced to a micrometric to nanometric level. In an aspect in which a laser beam is used, a finely shaped gel object can be produced with high accuracy.

As a method for producing a gel having a fine three-dimensional shape, a technology of producing a gel by UV-irradiation using a synthetic polymer and a photopolymerization initiator has been conventionally studied. However, many photopolymerization initiators have a risk of cytotoxicity, and a product resulting from photodegradation also carries the same risk. From the viewpoint of safety in the application to an organism such as a human body, apprehension has been pointed out about the application to a cell culture use and medical use. A material derived from an organism, for example, an extracellular matrix has various meaningful characteristics in an organism, for example, the scaffolding of a cell, the maintenance of a cell function, biocompatibility, and biodegradability. The material derived from an organism is commercially available in a variety of products, and the costs of production and procurement are reasonable. Also from such a viewpoint, the material derived from an organism is used as the biocompatible polymer.

A gel precursor composition and a method for producing a gel according to the present disclosure are based on technologies that inhibit the risk of cytotoxicity in a gel and a process of producing a gel, provide a two-dimensional or three-dimensional gel with high resolution and high accuracy, and achieve an economical efficiency together. One example of the contents of the present disclosure is, for example, a method for producing a gel, the method including a process in which a gel precursor composition containing gelatin, calcium-ion-dependent transglutaminase, and caged calcium is irradiated with a laser beam. Materials for the gel precursor composition, tools/instruments/devices for obtaining the gel precursor composition, and tools/instruments/devices for performing the method for producing a gel can all be produced and easily available. Because of its usefulness and economical efficiency, the present technology can be expected to be industrially applied. Transglutaminase can crosslink a biocompatible polymer under a mild environment in an organism to produce a gel, can also be used not only in vitro, for example, in a culture container but also in situ in an organism, and thus, can be expected to be widely applied to medical care.

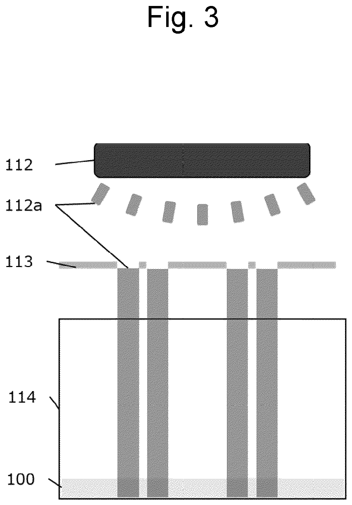

FIG. 3 is a schematic diagram of one aspect in which, using a photomask member, an uncrosslinked polymer contained in a gel precursor composition is crosslinked to produce a gel.

In the aspect illustrated in FIG. 3, the gel precursor composition 100 is uniformly disposed on the bottom face of a gel-precursor-composition-retaining container 114. The whole upper face of the gel precursor composition 100 is irradiated with irradiating light 112a from a light source 112, instead of the laser beam 111 in FIG. 1. A photomask member 113 is disposed between the light source 112 and the gel precursor composition 100. The photomask member 113 has holes opened at predetermined positions, and allows the irradiating light 112a to pass through the holes at the positions. The gel precursor composition 100 in and near the area irradiated with the irradiating light 112a is changed to the gel 100a.

In the present aspect, the irradiating light 112a causes a concentration distribution in the enzyme-activating substance released from the caged compound, and thus, the enzyme-activating substance is diffused, causing the range of activation of the enzyme to vary spatially and temporally. Progress in the crosslinking causes the elastic modulus of the gel to vary from moment to moment, and thus, it is preferable to design the concentration of the enzyme-activating substance in the gel precursor composition 100 and the concentration of the caged compound so as to maintain the enzyme activation time that affords a desired elastic modulus. In the same manner, there is the apprehension that the diffusion of the enzyme-activating substance causes gelation to progress in an unintended range. After studying a desired resolution and accuracy, it is possible to design various concentrations such that the concentration of the enzyme-activating substance falls below the enzyme-activating threshold after a given degree of diffusion, and to terminate a reaction by washing or the like after a given time.

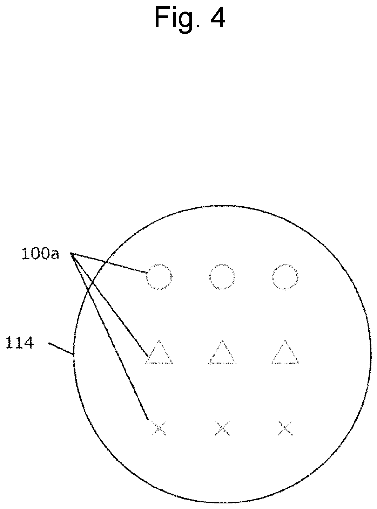

FIG. 4 is an example of a gel 100a obtained by gelation of a gel precursor composition 100 utilizing the photomask member 113 illustrated in FIG. 3, and having desired holes. After irradiation with the irradiating light 112a, portions (the gel precursor composition 100) that have not gelated can be removed, for example, by suction or washing with water, PBS, or the like. The gel 100a remains at the positions where the gel precursor composition 100 has gelated on the bottom face of the gel-precursor-composition-retaining container 114. Thus, gels having a desired shape or pattern can be obtained. Compared with a laser beam, the present method can simultaneously irradiate a wide area with light, and afford a large amount of gel in shape, and thus, yields high productivity. Accordingly, the method is effective in producing a large amount of gel having a specific shape or pattern. A three-dimensional shape can also be obtained as follows: after the gel 100a is produced, the gel precursor composition 100 is added; and the gel precursor composition 100 is allowed to gelate in the same manner, so that the gel 100a is layered.

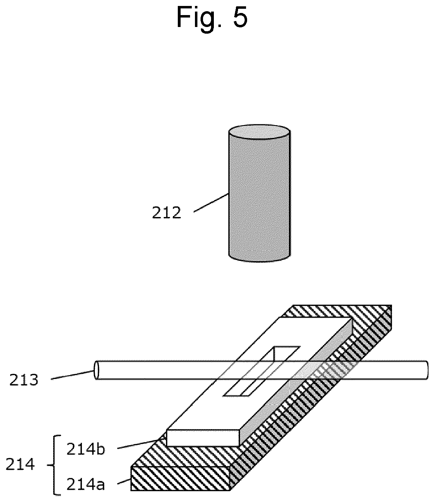

FIG. 5 is a schematic view illustrating Example of gelation of a gel precursor composition. The details will be described in Examples below. In Example, a light source used to give light for degrading a caged calcium was an ultraviolet LED the radiated light from which had a wavelength of 365 nm. That is, the irradiating light (light for degrading a photoresponsive compound) was UV-A in Examples, but may be visible light or the like that carries a lower risk of cytotoxicity, as long as the light has a wavelength capable of degrading a caged calcium to be used. Caged calcium that is degraded by visible light will undesirably be degraded in the environment of a general experiment, and thus, a black-out curtain or the like is desirably prepared in some cases. The caged calcium and the light source can be suitably selected taking into account cytotoxicity, a desired amount of irradiation, the cost for environmental arrangement, and the like in addition to the capability to cage and release a calcium ion.

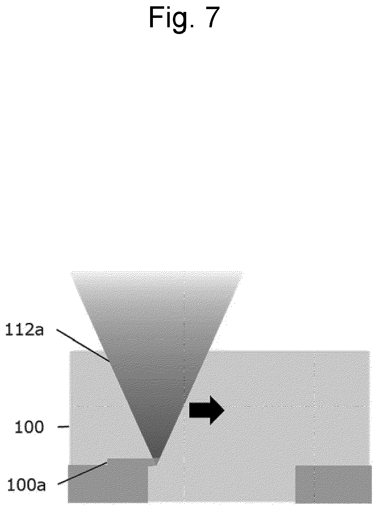

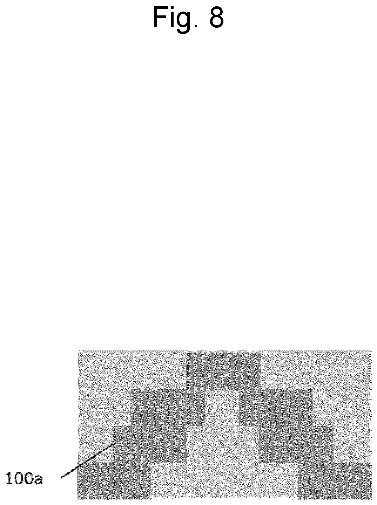

FIG. 7 and FIG. 8 are schematic diagrams of one aspect in which an uncrosslinked polymer contained in a gel precursor composition is crosslinked to produce a gel in an arbitrary three-dimensional shape.

FIG. 7 is a schematic diagram of a process in which a gel having an arbitrary three-dimensional shape is produced by irradiating a gel precursor composition with light. In the present aspect, the gel precursor composition 100 is a physical gel having a three-dimensional shape, and can reversibly change between a gel and a sol in accordance with a temperature, a pH, and the like. The three-dimensional shape is, for example, a column or a cube. The irradiating light 112a is condensed using a lens or the like, and have an amount of irradiation which can degrade a caged compound at and around the focus. Accordingly, except at and around the focus, the caged compound does not degrade, and gelation does not start. First, a desired pattern is scanned from the bottom face side to obtain a desired gel 100a. Next, the focus of the irradiating light 112a is moved vertically upward to obtain, in the same manner, a gel 100a having a desired pattern.

FIG. 8 is an example of a gel having a hollow shape. In a case where a gel having a hollow or bridging shape is produced, and where a gel precursor composition in liquid form is utilized, a gel in a gel-forming process will be deformed and collapsed by gravity or the like in some cases, thus failing to acquire a shape. Because of this, a supporting member is usually used, but this supporting member is finally removed. In a case where forming the gel having a desired shape is followed by removing the supporting member, a large external force is applied, and thus, such a supporting member is preferably not used from the viewpoint of the mechanical strength of the gel. Utilizing an additional member generally increases the complicatedness of the operation, also increases the risk of cytotoxicity and apprehension about safety, and hence, is desirably avoided when possible.

In the aspect illustrated in FIG. 7 and FIG. 8, the gel precursor composition 100 is a physical gel, thus has the function of a supporting member together, and hence, can yield the gel 100a having a desired shape. The uncrosslinked polymer that has not gelated can be easily removed by virtue of a change in the temperature and a change in the pH. Examples of such a material include gelatin. It is known that gelatin generally undergoes sol-gel transition at approximately 30°C. The activity of an enzyme is much changed by a change in the temperature, and depending on the temperature, an enzyme will be deactivated. At approximately 20°C, the maintenance of the enzymic activity of transglutaminase, which is one example of an enzyme, and the physical gelation of gelatin can be achieved simultaneously, allowing the application of the present technology. Other than gelatin, a material that undergoes sol-gel transition may be used, or a mixture of a plurality of materials may be used.

### EXAMPLES

Below, the present embodiment will be described with reference to Examples, and the present disclosure is not limited to these Examples.

### [Example 1]

### (Gelation of Gel Precursor Composition Using Photomask Member)

Using the following method, a gel precursor composition is allowed to gelate, producing a gel.

An aqueous solution of DM-NITROPHEN^{™} (a chelator for a caged calcium) from MERCK and an aqueous calcium chloride solution were mixed to prepare an aqueous solution containing caged calcium (calcium-bound DM-NITROPHEN) and a calcium ion.

Pig skin gelatin (Type A) 500G [High Jelly Strength] (a biocompatible polymer) manufactured by Nitta Gelatin Inc. and calcium-dependent transglutaminase (TG2) (Guinea pig liver transglutaminase from Zedira GmbH) (a crosslinking agent) were dissolved in pure water, and the resulting solution was mixed with an aqueous solution containing caged calcium (calcium-bound DM-NITROPHEN) and a calcium ion to obtain a gel precursor composition.

TG2 is activated when the concentration of the calcium ion exceeds a given value, and accordingly, TG2 is not activated when the gel precursor composition is prepared. Hence, the amount of the above-described DM-NITROPHEN^{™} and the amount of the calcium chloride were adjusted in such a manner that TG2 was activated when the below-described ultraviolet irradiation photodegrades the caged calcium to supply the calcium ion into a system.

The gel-precursor-composition-retaining container 214 illustrated in FIG. 5 includes a slide glass 214a as a mold frame and a silicone rubber 214b. Part of the silicone rubber 214b had a hole, and the gel precursor composition was injected through this hole. The size of the hole in the silicone rubber had a short side of 4 mm, a long side of 8 mm, and a depth of 0.5 mm.

A light source used to give light for degrading caged calcium was an ultraviolet LED212 (ULEDN-102CT manufactured by NS-Lighting Corporation) capable of radiating ultraviolet rays at 365 nm. As a photomask member, wood 213 having a sufficiently low UV transmittance was used. Wood 213 prepared had a shape capable of covering the hole having a short side of 4 mm and a long side of 2 mm.

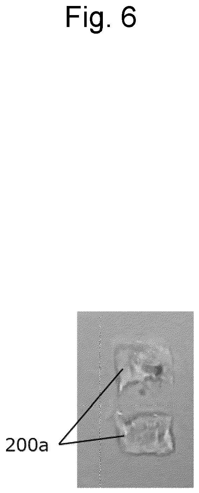

When the gel precursor composition was irradiated with ultraviolet rays, gelatin hydrogel 200a was produced at each of the upper and lower positions irradiated with the ultraviolet rays as in FIG. 6. However, the gel was not produced in the central portion, which was prevented by the wood 213 from ultraviolet rays, and the gel precursor composition was separable and removable at the interface. The gap portion between the gelatin hydrogels 200a in the upper and lower portions respectively was approximately 2.5 mm, was approximately 0.5 mm larger than 2 mm, the size of the masking wood, and gave a difference at a level smaller than millimeters. The above-described results have revealed that the present method for producing a gel can shape a gel with at least a submillimeter or smaller level of accuracy.

The upper limits and lower limits of the ranges of values described in the present specification can be arbitrarily combined to define a preferable range. For example, any upper limit and any lower limit of the ranges of values can be combined to define a preferable range. Any upper limits of the ranges of values can be combined to define a preferable range. Any lower limits of the ranges of values can be combined to define a preferable range.

### Description of the Related Art

### [Patent Literature]

[Patent Literature 1] Japanese Translation of PCT International Application Publication No. JP-T-2022-544752

## Claims

1. A gel precursor composition including:
a biocompatible polymer;
a crosslinking agent to be activated by a specific substance; and
a photoresponsive compound bound to the specific substance,
wherein the biocompatible polymer is a material derived from an organism,
wherein the crosslinking agent is a calcium-dependent enzyme that has any activity or no activity, depending on the calcium-ion concentration,
wherein the specific substance is a calcium ion,
wherein the photoresponsive compound is caged calcium, and
wherein the biocompatible polymer is a polymer that undergoes temperature-responsive sol-gel transition.

2. The gel precursor composition according to claim 1, wherein the biocompatible polymer is at least one polymer selected from gelatin and collagen.

3. The gel precursor composition according to claim 1, wherein the crosslinking agent is calcium-dependent transglutaminase.

4. The gel precursor composition according to claim 1, for use in the production of a gel to be used for a cell culture base material or a therapeutic base material.

5. A method for producing a gel comprising irradiating the gel precursor composition according to claim 1 with light to allow the gel precursor composition to gelate, the light having the capability to cause the photoresponsive compound to release the specific substance into a system.

6. The method for producing a gel according to claim 5, wherein the gelation is performed in the presence of at least one selected from a buffer and a pH adjustor.

7. The method for producing a gel according to claim 5, wherein the gelation is followed by adding at least one selected from: an inhibitor for the crosslinking agent; and a chelator for the specific substance.

## Patentansprüche

1. Gelvorläuferzusammensetzung, die einschließt:
ein biokompatibles Polymer;
ein Vernetzungsmittel, das durch eine spezifische Substanz zu aktivieren ist; und
eine photoresponsive Verbindung, die an die spezifische Substanz gebunden ist,
wobei das biokompatible Polymer ein aus einem Organismus abgeleitetes Material ist,
wobei das Vernetzungsmittel ein calciumabhängiges Enzym ist, das in Abhängigkeit von der Calciumionenkonzentration eine Aktivität oder keine Aktivität aufweist,
wobei die spezifische Substanz ein Calciumion ist,
wobei die photoresponsive Verbindung verkapseltes Calcium ist und
wobei das biokompatible Polymer ein Polymer ist, das einen temperaturabhängigen Sol-Gel-Übergang durchläuft.

2. Gelvorläuferzusammensetzung nach Anspruch 1, wobei das biokompatible Polymer mindestens ein Polymer ist, das aus Gelatine und Kollagen ausgewählt wird.

3. Gelvorläuferzusammensetzung nach Anspruch 1, wobei das Vernetzungsmittel calciumabhängige Transglutaminase ist.

4. Gelvorläuferzusammensetzung nach Anspruch 1, zur Verwendung bei der Erzeugung eines Gels, das für ein Zellkultur-Basismaterial oder ein therapeutisches Basismaterial verwendet werden soll.

5. Erzeugungsverfahren eines Gels, umfassend Bestrahlen der Gelvorläuferzusammensetzung nach Anspruch 1 mit Licht, um zu ermöglichen, dass die Gelvorläuferzusammensetzung geliert, wobei das Licht die Fähigkeit aufweist, die photoresponsive Verbindung zum Freisetzen der spezifischen Substanz in ein System zu veranlassen.

6. Erzeugungsverfahren eines Gels nach Anspruch 5, wobei die Gelierung in der Gegenwart mindestens eines durchgeführt wird, ausgewählt aus einem Puffer und einem pH-Einsteller.

7. Erzeugungsverfahren eines Gels nach Anspruch 5, wobei die Gelierung von Hinzufügen mindestens eines gefolgt wird, ausgewählt aus: einem Inhibitor für das Vernetzungsmittel; und einem Chelatbildner für die spezifische Substanz.

## Revendications

1. Composition de précurseur de gel comprenant :
un polymère biocompatible ;
un agent de réticulation à activer par une substance spécifique ; et
un composé photosensible lié à la substance spécifique,
dans laquelle le polymère biocompatible est un matériau dérivé d'un organisme,
dans laquelle l'agent de réticulation est une enzyme calcium-dépendante qui présente une activité ou aucune activité, selon la concentration en ions calcium,
dans laquelle la substance spécifique est un ion calcium,
dans laquelle le composé photosensible est du calcium piégé, et
dans laquelle le polymère biocompatible est un polymère qui subit une transition sol-gel thermosensible.

2. Composition de précurseur de gel selon la revendication 1, dans laquelle le polymère biocompatible est au moins un polymère choisi parmi la gélatine et le collagène.

3. Composition de précurseur de gel selon la revendication 1, dans laquelle l'agent de réticulation est une transglutaminase calcium-dépendante.

4. Composition de précurseur de gel selon la revendication 1, pour être utilisée dans la production d'un gel destiné à servir de matériau de base pour la culture cellulaire ou de matériau de base thérapeutique.

5. Procédé de production d'un gel comprenant l'irradiation de la composition de précurseur de gel selon la revendication 1 avec de la lumière pour permettre à la composition de précurseur de gel de se gélifier, la lumière présentant la capacité à amener le composé photosensible à libérer la substance spécifique dans un système.

6. Procédé de production d'un gel selon la revendication 5, dans lequel la gélification est réalisée en présence d'au moins un élément choisi parmi un tampon et un régulateur de pH.

7. Procédé de production d'un gel selon la revendication 5, dans lequel la gélification est suivie de l'ajout d'au moins un élément choisi parmi : un inhibiteur de l'agent de réticulation ; et un chélateur de la substance spécifique.
